# EUROPEAN PATENT APPLICATION

(11) **EP 4 431 450 A1**
(43) Date of publication of application: **18.09.2024**
(21) Application number: 23161816.6
(22) Date of filing: 14.03.2023
(51) Int. Cl.: B82Y 5/00, C12Q 1/6869

(54) **SUPPORTER FOR SEQUENCING TARGET NUCLEIC ACID STRANDS**

(71) Applicant: Leica Microsystems CMS GmbH, 35578 Wetzlar (DE)
(72) Inventor: Alsheimer, Dr. Soeren, 35578 Wetzlar (DE)
(74) Representative: Schaumburg und Partner Patentanwälte mbB

(57) **Abstract**

A supporter (104) for sequencing a target nucleic acid strand (100) of a biological sample (102) comprises an amplification structure configured for clonal amplification of a template nucleic acid strand (640) formed from at least a portion of the target nucleic acid (100) itself or from a nucleic acid strand complementary thereto, wherein the amplification structure is formed from a nanoscale scaffold (108) sized for in-situ clonal amplification of the template nucleic acid strand (640) within the biological sample (102).

## Description

### Technical field

The invention relates to a supporter for sequencing target nucleic acid strands of a biological sample. Further, the invention relates to a support network comprising a plurality of supporters and a method for sequencing target nucleic acid strands.

### Background

Next generation sequencing (NGS) refers to several high-throughput DNA sequencing methods that have strongly impacted progress in life sciences. Prior to the advent of the first NGS technology developed by Illumina, DNA sequencing was primarily performed using Sanger sequencing. A typical Sanger sequencer generates up to 384 sequences of up to 700 nucleotides length relying on what is known as terminator chemistry, i.e. sequencing by synthesis (SBS) using a low concentration of terminator nucleotides that are fluorescently labeled to identify the nucleobase and lack a 3'- OH group. Therefore, the terminator nucleotides terminate DNA synthesis of a strand in which they are incorporated.

Illumina sequencing and many other NGS methods are also based on sequencing by synthesis, fluorescent labeling, and optical readout for base calling. However, these methods achieve massively parallel sequencing reading millions to billions of nucleic acid fragments in parallel, which has allowed researchers to sequence entire genomes. For this reason, NGS has become a key platform for variety of applications ranging from genome sequencing for genetics and genomics, genome-wide association studies (GWAS), transcriptional profiling (RNAseq), and more recently also for single cell multi-omics.

While NGS-based methods are very powerful, they all rely on extracting nucleic acids from cells prior to sequencing. For example, in Illumina sequencing, which may also be referred to as paired-end sequencing, DNA is isolated from the cells of the biological sample following to lysis of the cells, then fragmented and ligated with corresponding primers, and finally flushed into a lane of a flow cell, where the ligated fragments are captured on complementary oligonucleotides that are bound on the bottom of the flow cell. After fragment capture, each fragment is amplified in a process known as bridge amplification thereby generating a clone of fragment. This basically serves to amplify the signal that is being generated during the sequencing-by-synthesis step to detectable levels. As a result, spots are created which are bright enough to allow reliable base calling. In such a case, the sequencing reaction occurs in a cyclical process in which labeled terminator nucleotide are incorporated, readout, and then terminator and label are removed. In general, applied chemistry uses either four colors to discern the four different nucleobases. Alternatively, only two colors may be used, as in a new system developed by Illumina (called NovaSeq), or even single-color chemistry can be used.

Since conventional NGS methods require a removal of nucleic acids from their natural biological environment prior to sequencing, a spatial context that would allow nucleic acids to be studied in terms of their spatial relationship within the biological environment is lost. This limits applications in spatial biology that aim to study nucleic acids in their unique context within individual cells and tissues.

### Summary

It is an object to provide a supporter which allows sequencing of target nucleic acid strands in their natural biological environment.

The afore-mentioned object is achieved by a supporter for sequencing target nucleic acid strands of a biological sample according to claim 1. Advantageous embodiments are defined in the dependent claims and the following description.

The supporter disclosed herein allows sequencing of a target nucleic acid strand of a biological sample. The supporter comprises an amplification structure configured for clonal amplification of a template nucleic acid strand formed from at least a portion of the target nucleic acid itself or from a nucleic acid strand complementary thereto. The amplification structure is formed from a nanoscale scaffold which is sized for in-situ clonal amplification of the template nucleic acid strand within the biological sample.

The supporter may be configured to form a three-dimensional structure that allows to perform next generation sequencing (NGS) in 3D rather than in 2D such as in conventional NGS approaches that uses a bottom of a flow cell for clonal amplification. Thus, it is not required to extract the target nucleic acid strands from the biological sample before sequencing is performed. In particular, no cell lysis is necessary.

To be sized for in-situ clonal amplification, the largest spatial extent of the nanoscale scaffold may be in a range from 0.1 nm to 200 nm, preferably from 1 to 100 nm and even more preferably from 10 nm to 50 nm.

In a preferred embodiment, the nanoscale scaffold is a DNA nanotechnology-based structure. For example, the nanoscale scaffold may made from DNA tiles, DNA origami, or DNA bricks. The use of DNA bricks is particularly advantageous. DNA bricks are shorter length oligonucleotides that have overlapping hybridising stretches in order to bind to each other and assemble a backbone nanoscale scaffold. DNA bricks can be readily introduced into the sample where they first assemble to form the nanoscale scaffold.

Preferably, the nanoscale scaffold comprises a plurality of adapter oligonucleotides of at least two types configured to attach primer oligonucleotide portions of at least two types for clonal bridge amplification of the template nucleic acid strand. Those adapter oligonucleotides are available in wide variety to ensure effective bridge amplification on the nanoscale scaffold.

For example, the nanoscale scaffold may be configured for clonal amplification of a cDNA strand. In this case, a cDNA strand is used as the template nucleic acid strand which is amplified on the nanoscale scaffold. However, it is also possible to amplify the target nucleic acid strand itself before sequencing its copies.

In a preferred embodiment, the nanoscale scaffold is configured to be directly bound to or assembled on the target nucleic acid to be sequenced. Thus, it is ensured that the nanoscale scaffold is located exactly where it is needed for subsequent clonal amplification. As a result, optimal spatial correlation is achieved.

According to another aspect, a support network is provided which comprises a plurality of supporters as described above. In contrast to a flow cell that is conventionally used in NGS approaches for amplification, the support network enables clonal amplification without cell lysis. Accordingly, the support network can be used in a platform for assays that allow DNA, RNA or even protein sequences to be directly read inside a biological sample.

According to a preferred embodiment, the support network comprises a stabilization structure configured to interconnect the supporters. Such a structure serves to make the support network more stable which is achieved by suitably connecting the supporters with each other.

Preferably, the stabilization structure comprises a polymer network or a gel matrix. By using such a polymer network or gel matrix, a 2D or 3D array of sufficient regularity can be established within the sample. For example, the array may be formed from hybrid nucleic acid-polymers such as peptide-nucleic acids or PED nucleic acids, wherein the nucleic acid portion may be an oligonucleotide, a DNA tile, a DNA brick, or a DNA origami.

In a preferred embodiment, the stabilization structure is configured to be cross-linked to cellular constituents of the biological sample. Such a cross-link makes the support network even more stable. In addition, the support network is enabled to be expanded together with the sample and is thus particularly suitable in the field of expansion microscopy.

Preferably, the supporters are formed from building blocks pre-assembled outside the biological sample. In this case, the supporters are introduced into the sample where they interconnect to form the support network.

According to an alternative embodiment, each supporter comprises multiple sub-units which are configured for in-situ self-assembly within the biological sample. Also the nanoscale scaffold itself may be formed from such sub-unit such as DNA bricks.

According to another embodiment, a method is provided for sequencing target nucleic acid strands of a biological sample using the support network as described above. The method comprises the following steps: providing the support network within the biological sample; adsorbing the template nucleic acid strands on the amplification structures of the support network; performing in-situ clonal amplification of each template nucleic acid strand adsorbed on the amplification structure within the biological sample to generate a cluster which includes a plurality of copies of the template nucleic acid strand; and performing sequencing on each cluster.

According to a preferred embodiment, the method comprises a step of adding optically detectable labels to the copies included in the respective cluster, and a step of optically reading out the labels.

Preferably, the method further comprises a step of physically expanding the biological sample to increase spatial resolution of the optical readout.

### Short Description of the Figures

Hereinafter, specific embodiments are described referring to the drawings, wherein:
- Figure 1: is a schematic diagram illustrating an arrangement that comprises a biological sample and a support network for sequencing target nucleic acid strands within the sample according to an embodiment;
- Figure 2: is a schematic diagram illustrating the arrangement that comprises the biological sample and the support network to another embodiment;
- Figure 3: is a schematic diagram illustrating the arrangement that comprises the biological sample and the support network to another embodiment;
- Figure 4: is a schematic diagram illustrating the arrangement that comprises the biological sample and the support network to another embodiment;
- Figure 5: is a schematic diagram illustrating the arrangement that comprises the biological sample and the support network to another embodiment;
- Figures 6A: a schematic diagram illustrating a step of a process for clonal amplification using the support network to an embodiment;
- Figures 6B: a schematic diagram illustrating a further step of the process for clonal amplification;
- Figures 6C: a schematic diagram illustrating a further step of the process for clonal amplification;
- Figures 6D: a schematic diagram illustrating a further step of the process for clonal amplification;
- Figures 6E: a schematic diagram illustrating a further step of the process for clonal amplification;
- Figures 6F: a schematic diagram illustrating a further step of the process for clonal amplification;
- Figures 6G: a schematic diagram illustrating a further step of the process for clonal amplification;
- Figures 6H: a schematic diagram illustrating a further step of the process for clonal amplification;
- Figure 7: is a schematic diagram showing different exemplary configurations of a nanoscale scaffold usable in the support network; and
- Figure 8: is a schematic diagram illustrating a spatial distribution of cDNA clusters generated on the nanoscale scaffolds inside a sample volume.

### Detailed Description

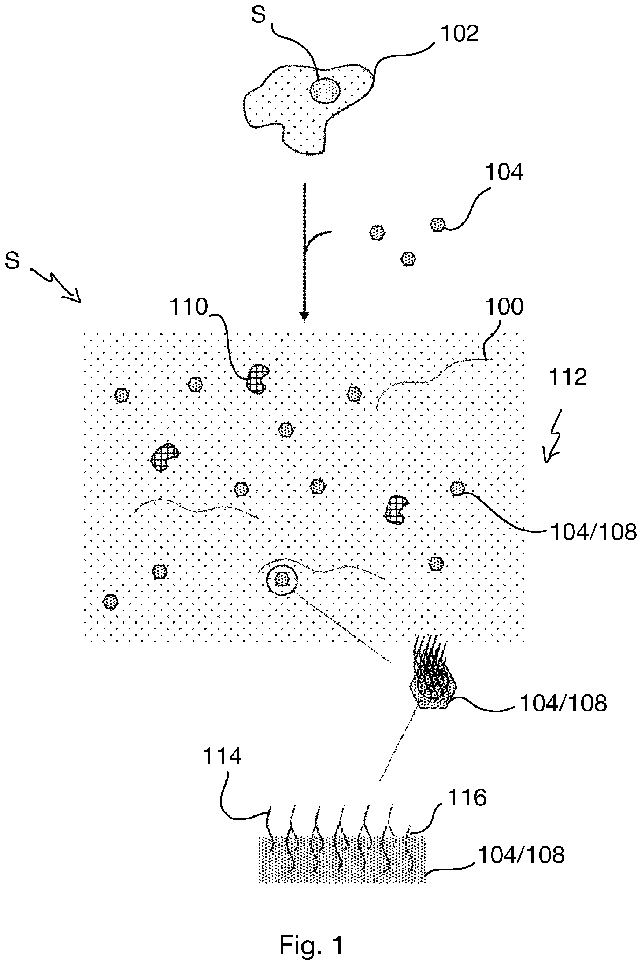

Figure 1 is a schematic diagram illustrating an arrangement that enables high through-put NGS sequencing of target nucleic acids 100 in a biological sample 102. In particular, the arrangement allows sequencing to be performed in-situ which means that sequencing does not require removing the target nucleic acid strands 100 from the biological sample 102.

For example, the sample 102 may be a biological cell into which a plurality of supporters 104 can be introduced. A top portion of Figure 1 shows the biological sample 102 in its entirety, while a middle portion of Figure 1 shows an enlarged section S of the sample 102. To enable infusion of the supporters 104 into the biological sample 102 without compromising its integrity, each supporter 104 comprises an amplification structure that is formed from a nanoscale scaffold 108. Each nanoscale scaffold 108 is configured for in-situ clonal amplification of the nucleic acid strands 100 within the intact sample 102 as illustrated in a lower portion of Figure 1. Each nanoscale scaffold 108 may be made from a DNA nanotechnology-based structure. For example, each nanoscale scaffold 108 is formed from a DNA tile or a DNA origami. Alternatively, the nanoscale scaffold may comprise a plurality of DNA bricks.

According to the present embodiment, the nanoscale scaffold 108 has a size such that its maximum spatial extent is in a range from 0.1 nm to 200 nm, preferably from 1 nm to 100 nm, and more preferably from 10 nm to 50nm. The size of the supporters 104 is selected to allow, on the one hand, the supporters 104 to be readily infused into the biological sample 102. Thus, it is ensured that the biological sample 102 remains intact during infusion of the supporters 104 into the sample 102. Importantly, no cell lysis is required prior to sequencing.

On the other hand, the supporters 104 may be sized to be large enough to carry a sufficient number of oligonucleotides that enable effective clonal amplification of the target nucleic acid strands 100. For example, each nanoscale scaffold 108 may carry a plurality of first adapter oligonucleotides 114 and a plurality of second adapter oligonucleotides 116 as illustrated in the lower portion of Figure 1 by solid lines and dashed lines, respectively.

A suitable concentration of the supporters 104 is selected so that the supporters 104 are stochastically distributed in sample 102. Accordingly, the supporters 104 are present in the sample 102 in a number which is sufficiently high to ensure that, on average, for each target nucleic acid strand 100 one supporter 104 can be expected to be located near or directly on the target nucleic acid strand 100. Notably, the spatial relationships between the target nucleic acid strands 100 and other cellular constituents 110 such as proteins are preserved in the intact biological sample 102 as shown in Figure 1.

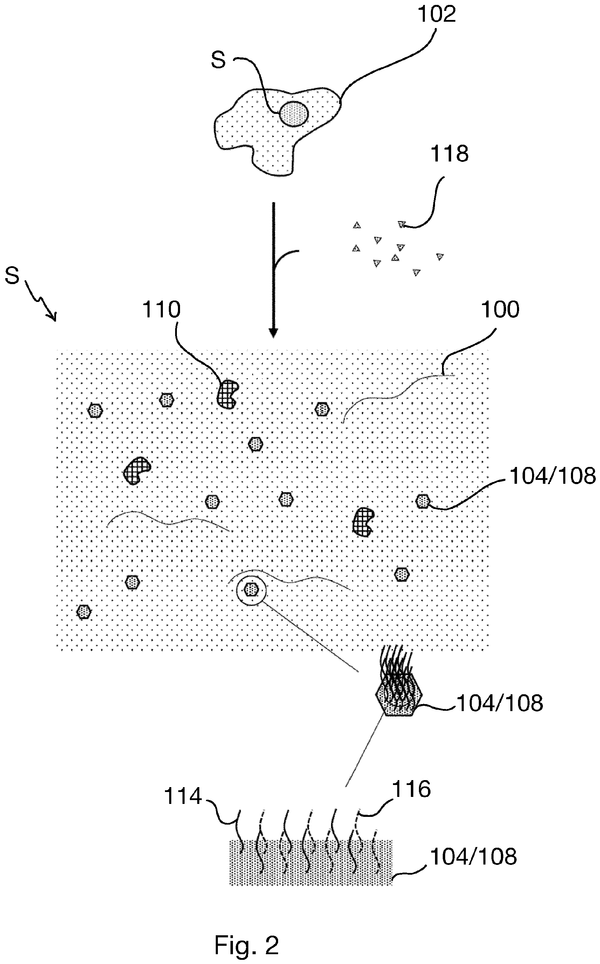

According to the embodiment shown in Figure 1, each nanoscale scaffold 108 may be formed as integral unit that is introduced into the sample 102. Alternatively, as shown in Figure 2, a plurality of scaffold sub-units such as DNA bricks 118 may be introduced into the sample 102 where they first assemble to form the nanoscale scaffold 108.

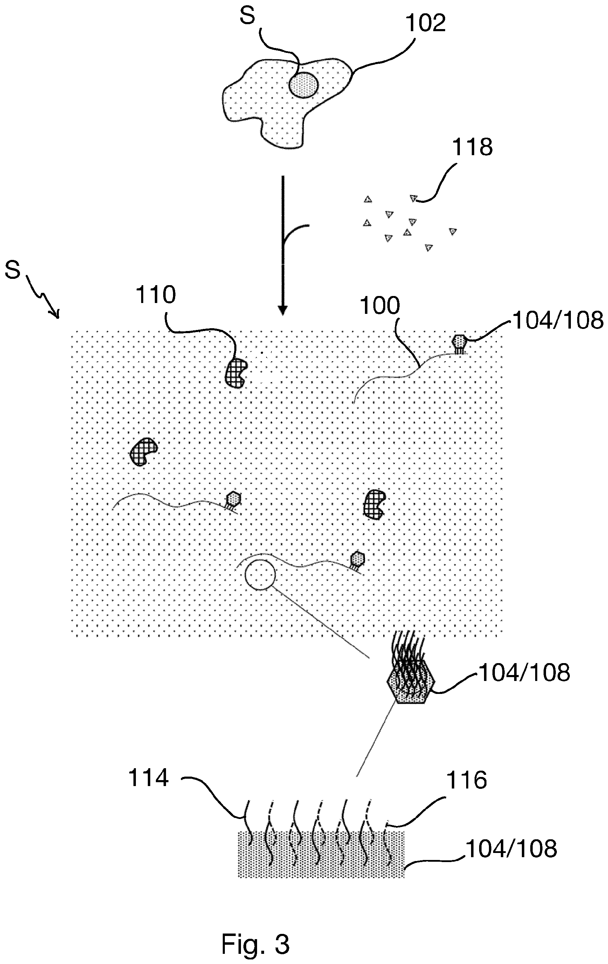

Although a reasonably uniform spatial distribution can be achieved by simply introducing the nanoscale scaffolds 108 into the sample 102 as shown in Figures 1 and 2, it may be desirable to further improve a correlation between the location of the respective target nucleic acid 100 and the location of a cDNA cluster that is created on the nanoscale scaffold 108 in a process of clonal amplification described below. A modification that serves this purpose is shown in Figure 3.

According to the embodiment shown in Figure 3, the DNA bricks 118 introduced into the sample 102 configured to be assembled on the target nucleic acid strands 100 to form the nanoscale scaffolds 108 right there. Thus, the nanoscale scaffolds 108 are generated at the locations of the target nucleic acid stands 100, i.e. at the precise locations where they are needed for subsequent clonal amplification. This may be achieved by incorporating the oligo-dT primer used for reverse transcription into the nanoscale scaffolds 108. In this way, the nanoscale scaffold 108 is then routed to the poly(A) tail of an mRNA. Alternatively, or in addition a nanoscale scaffold may be targeted to a specific set of mRNA or a specific genomic locus by using a sequence-specific primer extension of the nanoscale scaffold 108. This basically allows either routing of the nanoscale scaffolds 108 or their assembly on poly(A) tails or their desired locations. Furthermore, it is possible to route the nanoscale scaffolds 108 by connecting them to an affinity reagent like for example an antibody, nanobody or aptamer. The latter of which may be particularly preferred due its small size. An affinity reagent against poly(A) binding protein (PABP), cap binding protein (CPB), translation initiation factors may be used to route the nanoscale scaffolds 108 or their sub-units to messenger ribonucleoprotein (mRNP) complexes. Similarly, affinity reagents against certain histone modifications may target the nanoscale scaffolds 108 to certain epigenetic marks on the DNA like silenced loci or open chromatin. Affinity reagents against components of RNA polymerase may be used to target the nanoscale scaffolds 108 or their subunits to sites of active transcription.

As a result, optimal spatial correlation is achieved. In particular, a situation can be avoided where cDNA molecules are lost because they happen to be too far from the nearest nanoscale scaffold 108.

According to the embodiment shown Figure 3, each nanoscale scaffold 108 is assembled from multiple DNA bricks 118. However, it is also possible that the nanoscale scaffolds 108 are already present as integral units before being introduced into the sample 102. In this case, the nanoscale scaffolds 108 are configured to bind directly to the target nucleic acid strands 100 to achieve the intended spatial correlation.

According to the embodiments shown in Figures 1 to 3, the plurality of nanoscale scaffolds 108 form a structure which is substantially uniformly distributed within the biological sample 102. Taking into account the size of the nanoscale scaffolds 108 as specified above, it can be assumed that the nanoscale scaffolds 108 are fairly immobile in the sample 102. In other words, the nanoscale scaffolds 108 do not excessively diffuse within the sample 102. Therefore, the nanoscale scaffolds 108 can be expected to form a reasonably stable support network 112, although they are not interconnected.

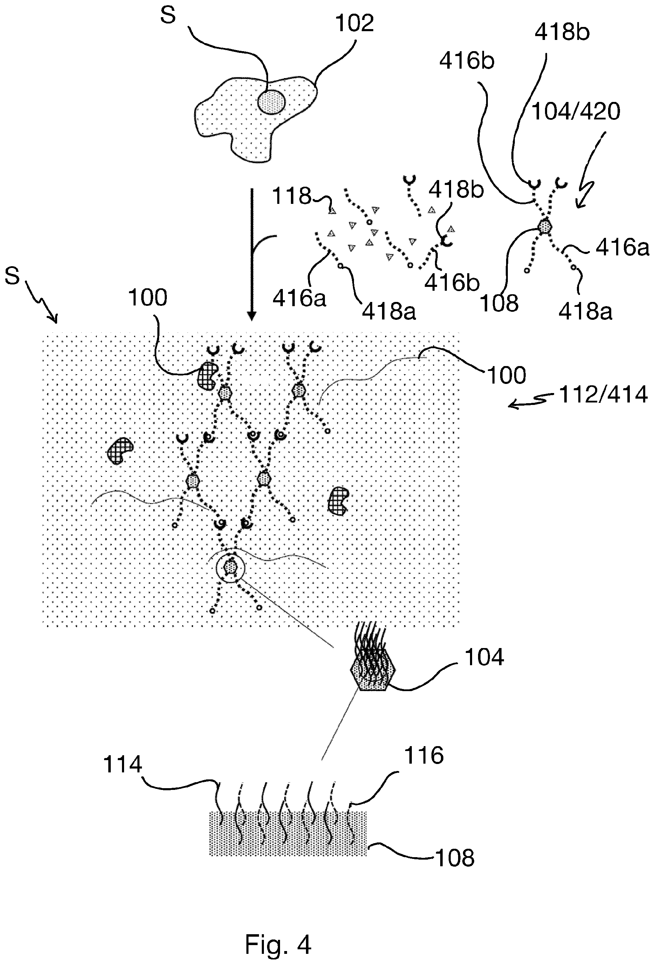

As a modification to the embodiments shown in Figures 1 to 3, the support network 112 of Figure 4 includes an additional stabilization structure 414 which serves to make the support network 112 even more stable by interconnecting the supporters 104. For example, the stabilization structure 414 may comprise a polymer network or a gel matrix.

For providing a stabilizing polymer network, each supporter 104 may have multiple polymer arms 416a, 416b of two different types extending from the nanoscale scaffold 108. The polymer arms 416a, 416b are functionalized to bind to each other. More specifically, a first type of polymer arms 416a may include a first affinity interactor 418a or reactive group located on a free end of the respective polymer arm 416a. Likewise, a second type of polymer arms 416b may include a second affinity interactor 418b or reactive group located on a free end of the respective polymer arm 416b. The first and second affinity interactors 418a, 418b or reactive group are configured to bind to each other for interconnecting the nanoscale scaffolds 108. Thus, the polymer network is created.

In addition or as an alternative, the affinity interactors may be replaced or augmented by chemical moieties configured to react with each other and/or components of the sample like for example proteins to establish covalent cross-links. A variety of cross-linking chemistries exists, which may be combined with spacers of varying length. Generally used cross-linking chemistries which may be used for this task include but are not limited to: NHS ester reaction with amine-residues (amine bond), maleimide reaction with sulfhydryl-residues (thioether bond), hydrazide reaction with aldehyde-containing molecules (hydrazone link), EDC coupling with carboxylate group (amine bond), click-chemistries such as BCN (bicyclo[6.1.0]nonyne with azide-tagged molecules, alkyne-azide, DBCO (dibenzocyclooctyne)-azide, TCO (trans-cyclooctene)-tetrazine.

As illustrated in the top portion of Figure 4, each supporter 104 may be formed from multiple sub-units which are configured be assembled only in-situ within the sample 102. The sub-units of each supporter 104 may be given by the nanoscale scaffold 108 that is introduced as an integral unit into the sample 102, and the polymer arms 416a, 416b. Alternatively, a plurality of DNA bricks 118 may be introduced into the sample 102 where they first assemble to form the nanoscale scaffold 108.

As further shown in Figure 4, the supporters 104 may also be formed from building blocks 420 which are pre-assembled outside the biological sample 102. Accordingly, each nanoscale scaffold 108 is already provided with its polymer arms 416a, 416b before being introduced as building block 420 into the sample 102. Within the sample 102, the polymer arms 416a, 416b of this building block 420 need only be connected to polymer arms of surrounding building blocks to form the stabilizing polymer network.

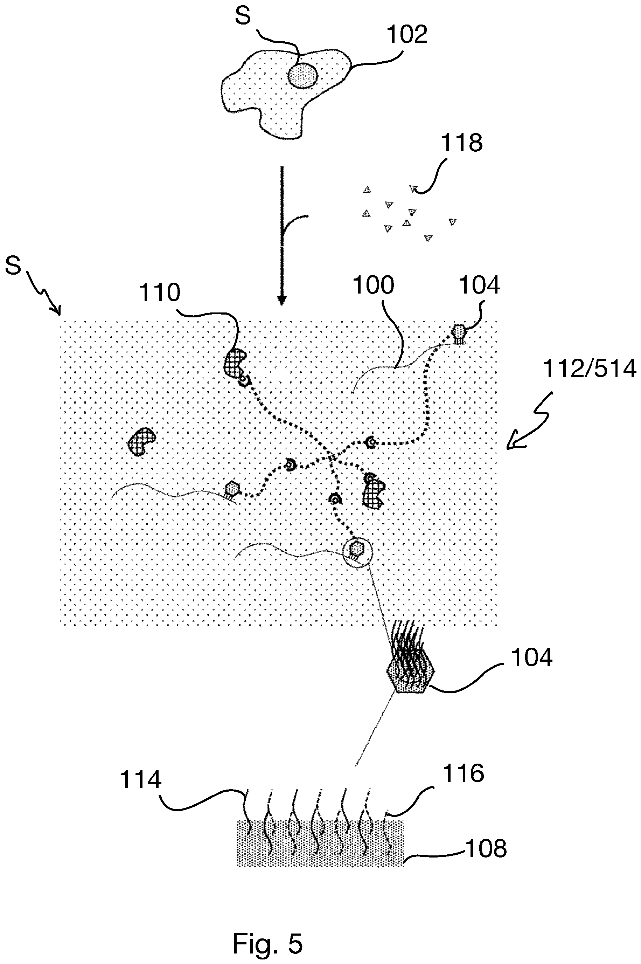

Figure 5 illustrates another embodiment in which the support network 112 comprises a modified stabilization structure 514. Compared to the stabilization structure 414 shown in Figure 4, the stabilization structure 514 of Figure 5 is further configured to be cross-linked to the cellular constituents 110 of the sample 102 such as proteins.

A variety of cross-linking chemistries exists, which may be combined with spacers of varying length. Generally used cross-linking chemistries which may be used for this task include but are not limited to: NHS ester reaction with amine-residues (amine bond), maleimide reaction with sulfhydryl-residues (thioether bond), hydrazide reaction with aldehyde-containing molecules (hydrazone link), EDC coupling with carboxylate group (amine bond), click-chemistries such as BCN (bicyclo[6.1.0]nonyne) with azide-tagged molecules, alkyne-azide, DBCO (dibenzocyclooctyne)-azide, TCO (trans-cyclooctene)-tetrazine.

Suitable spacers may be for example poly-ethylene glycol or maybe themselves based on nucleic acids.

Cross-linking the stabilization structure 514 to the cellular constituents 110 of the sample 102 further stabilizes the support network 112. In addition, the support network 112 scales with the biological sample 102 when the sample 102 is physically expanded as in expansion microscopy.

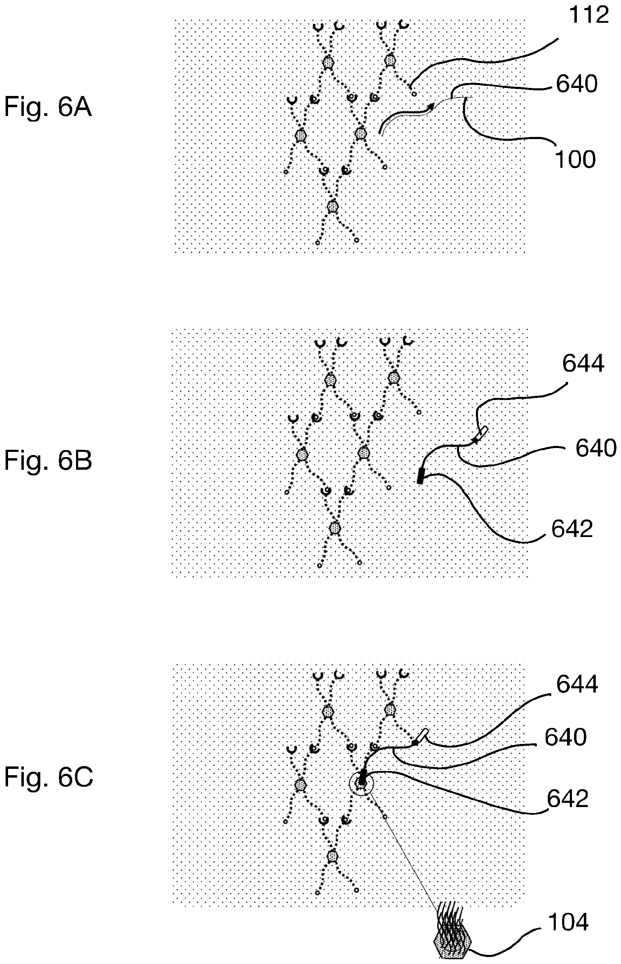

Figures 6A to 6H illustrate successive steps of a process for clonal amplification. This process is executed according to the principle of sequence by synthesis (SBS). However, instead of using a bottom of a flow cell as in conventional SBS approaches, the method disclosed herein employs the supporters 104 described above which allow clonal amplification within the sample 102 without cell lysis. As an example, it assumed that the clonal amplification is performed by means of the support network 112 shown in Figure 4.

Figure 6A shows a step in which a cDNA synthesis is performed on an mRNA strand which forms the target nucleic acid strand 100 that is to be sequenced. As a result, a template nucleic acid strand in form of a cDNA strand 640 is generated in close proximity to one of the nanoscale scaffolds 108 forming the support network 112.

During cDNA synthesis, two different primer oligonucleotides 642, 644 are added to end portions of the cDNA strand 640 as illustrated in Figure 6B.

Subsequently, the cDNA strand 640 is captured by the nearest nanoscale scaffold 108 as shown in Figure 6C. As described above, the nanoscale scaffold 108 carries the first and second adapter oligonucleotides 114, 116 which are complementary to the first and second primer oligonucleotides 642, 644 of the cDNA strand 640, respectively. In the example of Figure 6C, it is assumed that the first primer oligonucleotides 642 of the cDNA strand 640 hybridizes to one of the first adapter oligonucleotides 114 of the nanoscale scaffold 108 when the cDNA strand 640 is captured by the nanoscale scaffold 108.

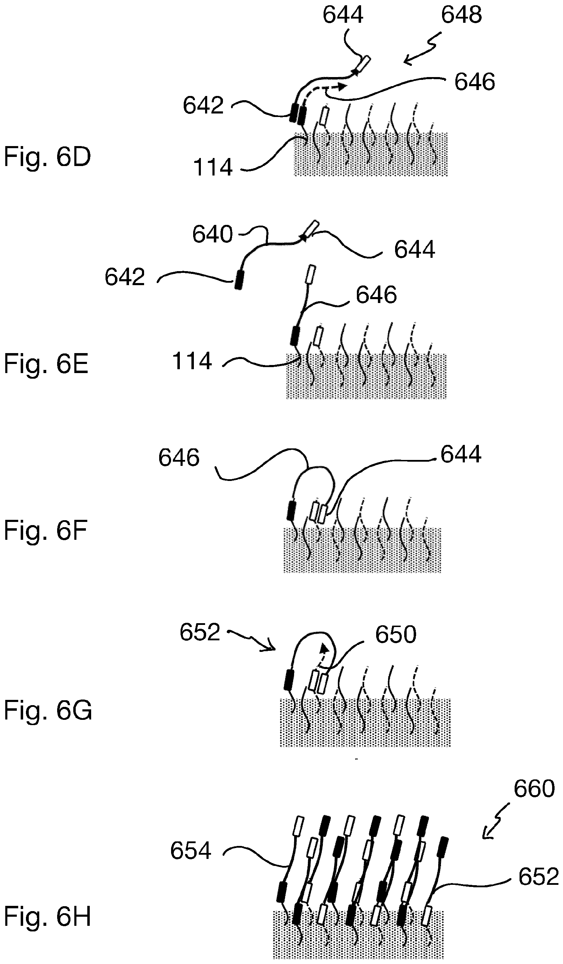

In a next step according to Figure 6D, a reverse strand 646 complementary to the hybridized cDNA strand 640 is generated on the nanoscale scaffold 108. As result, a duplex 648 is created in form of a double-stranded molecule consisting of the strands 640, 646, with strand 646 being covalently connected to the adapter oligonucleotide, which itself may be covalently or indirectly (e.g. via hybridization) connected to the nanoscale scaffold 108.

The duplex 648 is then denatured, and the original cDNA strand 640 is removed from the nanoscale scaffold 108 as shown in Figure 6E.

Figure 6F shows how the strand 646 is subsequently amplified on the nanoscale scaffold 108 by means of bridge amplification. In this step, the strand 646 bends over, and the second primer oligonucleotide 644 hybridizes to one of the second adapter oligonucleotides 116 carried on the nanoscale scaffold 108.

Then, as shown in Figure 6G, a strand 650 complementary to the strand 646 is generated. As result, a duplex 652 in form of a double-stranded bridge is created on the nanoscale scaffold 108. The duplex 652 is subsequently denatured and removed from the nanoscale scaffold 108.

By repeatedly performing this process, a plurality of single-stranded copies of the cDNA strand 640 originally captured by the nanoscale scaffold 108 are generated, as shown in Figure 6H, with these copies being formed from reverse strands 650 and forward strands 654. If desired, either the reverse strands 650 or the forward strands 654 can then be removed from the nanoscale scaffold 108.

As a result of the clonal amplification process, each nanoscale scaffold 108 of the support network on which the cDNA strand 640 is bound, a cluster 660 including the plurality of (forward or reverse or both forward and reverse) single-stranded copies of the cDNA strand 640 is obtained.

It is to be noted that the amplification process described above acts on the cDNA strand 640 forming a template nucleic acid strand derived from the mRNA strand 100 that represents the target nucleic acid strand to be sequenced.

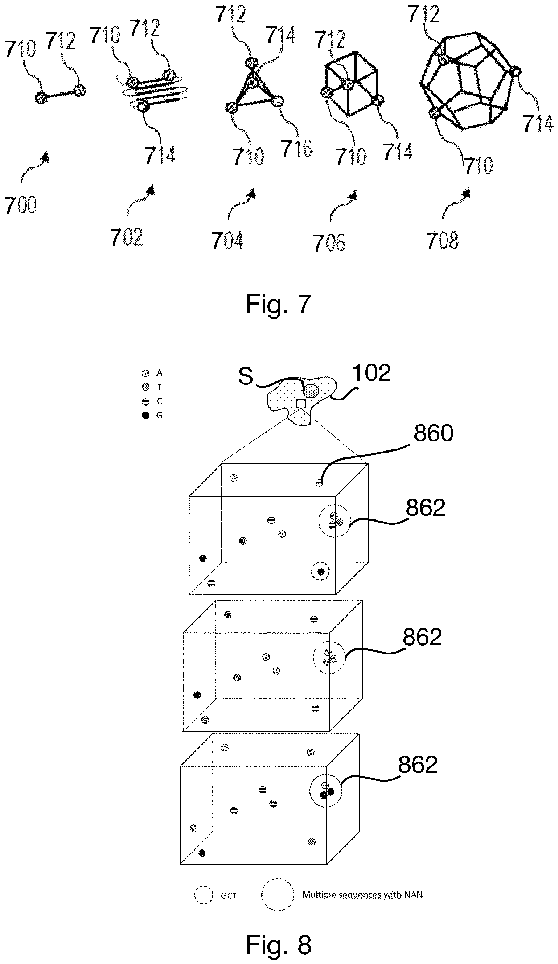

Figure 7 shows is a schematic diagram showing different exemplary configurations of the nanoscale scaffold 108 usable in the support network 112.

The configurations illustrated in Figure 7 are formed from nucleic acid backbones 700, 702, 704, 706, 708 with different geometries. Generally, the backbones 700 - 708 comprise nucleic acids. In particular, the backbones 700 - 708 may be DNA-origami based, which allows generating predetermined, stable two- and three-dimensional shapes. Further, this allows generating a plurality of attachment sites at predetermined positions along the backbones 700 - 708. The attachment sites are stretches of oligonucleotides that are unique and allow the hybridisation of complementary oligonucleotides, for example to attach optical labels to the backbones 700 - 708 at these predetermined positions.

Backbone 700 is linear or rod-like. It comprises a first orientation indicator 710 and a second orientation indicator 712. The orientation indicators 710, 712 may be used to determine the orientation, directionality or polarity of the backbone 700. The orientation indicators 710, 712 may comprise a dye, in particular a fluorescent dye, such as fluorescein or a fluorescent protein. In addition, the dye of the first orientation indicator 710 may have different characteristics than the dye of the second orientation indicator 712. The characteristics may include fluorescent emission characteristics, excitation characteristics or lifetime characteristics. This enables differentiating between the first and the second orientation indicators 710, 712 in an optical readout of the backbone 700, for example generated by a microscope, a cytometer, or an imaging cytometer. The orientation indicators 710, 712 are arranged spaced apart from each other. Preferably each orientation indicator 710, 712 is arranged on the backbone 700 at opposite ends. Thus, the first and second orientation indicators 710, 712 enable differentiating between a first end and a second end of the backbone 700. Ultimately, this enables determining the orientation, directionality or polarity of the backbone 700, for example from the first orientation indicator 710 on the first end to the second orientation indicator 712 on the second end.

The backbone 702 is sheet-like, which may be a large linear DNA molecule or an assembly of multiple DNA molecules. Sheet-like backbones may increase the number of available attachment sites substantially. In order to be able to determine the orientation of the backbone 702, a third orientation indicator 714 is provided.

Further geometries are possible, for example, the tetrahedral backbone 704, the cubic backbone 706, or the polyhedral backbone 708. These may comprise a fourth orientation indicator 716 in order to determine their orientation.

Figure 8 illustrates how multiple cDNA clusters 860 formed on the nanoscale scaffolds 108 may be distributed in a 3D volume 800 inside the sample 102. Each cDNA cluster 860 may be provided with optical labels of the same type, e.g. a distinct fluorescence dye. Thus, for each cDNA cluster 860, a spot may be detected by means of a readout device such as a microscope.

While the majority of cDNA clusters 860 can be expected to have a distance from each other that is larger than a lateral and/or axial resolution of the readout device, some of the cDNA clusters 860 may be colocalized in the same readout volume, the latter being defined by a point spread function (PSF) of an optical detection system of the readout device. Such a colocalization of cDNA clusters renders reading of the corresponding sequences more challenging.

Different strategies may be employed to read DNS sequences in colocalizing clusters. For example, (re)-priming sequencing reactions from starting primes belonging to multiple sets may be applied. In this way, a cluster containing three targets such a cluster indicated by reference sign 862 in Figure 8 may be read in three cycles, each cycle including a sequencing-by-synthesis step.

In case that a user performs the sequencing reaction only to read the identity of the underlying analyte or marker, then a partial sequence may be generated of the type NAN for example, wherein N stands for any nucleotide. In this example, the first and third sequencing-by-synthesis cycle of the readout volume containing three clusters did not provide clear results as the sequences were different in these positions (nevertheless A,T,C were read but could not be assigned to either sequence). The second cycle in this example, however, showed that all sequences have an A in this position. In this way a consensus sequence can be defined for the readout volume across all included clusters which may look like the sequence NANNNNGCN...., for example. This information may be sufficient to retrieve the possible analyte sequences by database comparison, using for example BLAST or a similar algorithm. In particular, this may yield sufficient statistical confidence in the deconvolution of the readout volume, if an appropriate strategy is used to decode the identities of the included analytes.

As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of Reference Signs

- 100: target nucleic acid strand
- 102: sample
- 104: supporter
- 108: nanoscale scaffold
- 110: cellular constituent
- 112: support network
- 114, 116: adapter oligonucleotide
- 118: DNA brick
- 414: stabilization structure
- 416a, 416b: polymer arm
- 418a, 418b: affinity interactor
- 420: building block
- 514: stabilization structure
- 640: cDNA strand
- 642,644: primer oligonucleotide
- 646: reverse strand
- 648,652: duplex
- 650, 654: strand
- 660: cluster
- 700 - 708: nucleic acid backbone
- 710-714: indicator
- 800: 3D volume
- 860,862: cluster
- S: section of sample

## Claims

1. A supporter (104) for sequencing a target nucleic acid strand (100) of a biological sample (102), comprising an amplification structure configured for clonal amplification of a template nucleic acid strand (640) formed from at least a portion of the target nucleic acid (100) itself or from a nucleic acid strand complementary thereto, wherein the amplification structure is formed from a nanoscale scaffold (108) sized for in-situ clonal amplification of the template nucleic acid strand (640) within the biological sample (102).

2. The supporter (104) according to claim 1, wherein the largest spatial extent of the nanoscale scaffold (108) is in a range from 0.1 nm to 200 nm.

3. The supporter (104) according to any one of the preceding claims, wherein the nanoscale scaffold (108) is a DNA nanotechnology-based structure.

4. The supporter (104) according to any one of the preceding claims, wherein the nanoscale scaffold (108) comprises a plurality of adapter oligonucleotides (114, 116) of at least two types configured to attach primer oligonucleotide portions (642, 644) of at least two types for clonal bridge amplification of the template nucleic acid strand (640).

5. The supporter (104) according to any one of the preceding claims, wherein the nanoscale scaffold (108) is configured for clonal amplification of a cDNA strand (640).

6. The supporter (104) according to any one of the preceding claims, wherein the nanoscale scaffold (108) is configured to be directly bound to or assembled on the target nucleic acid (100) to be sequenced.

7. A support network (112) comprising a plurality of supporters (104) according to one of the preceding claims.

8. The support network (112) according to claim 7, comprising a stabilization structure (414, 514) configured to interconnect the supporters (104).

9. The support network (112) according to claim 8, wherein the stabilization structure (414, 514) comprises a polymer network or a gel matrix.

10. The support network (112) according to claim 8 or 9, wherein the stabilization structure (514) is configured to be cross-linked to cellular constituents (110) of the biological sample (102).

11. The support network (112) according to any one of the claims 7 to 10, wherein the supporters (104) are formed from building blocks (420) pre-assembled outside the biological sample (102).

12. The support network (112) according to any one of the claims 7 to 10, wherein each supporter (104) comprises multiple sub-units (118, 416a, 416b) which are configured for in-situ self-assembly within the biological sample (102).

13. A method for sequencing target nucleic acid strands (100) of a biological sample (102) using the support network (112) according to any one of the claims 7 to 12, comprising the following steps:
providing the support network (112) within the biological sample (104),
adsorbing the template nucleic acid strands (640) on the amplification structures of the support network (112),
performing in-situ clonal amplification of each template target nucleic acid strand (640) adsorbed on the amplification structure within the biological sample (102) to generate a cluster (660) which includes a plurality of copies (650, 652) of the template nucleic acid strand (640), and
performing sequencing on each cluster (660).

14. The method according to claim 13, further comprising a step of adding optically detectable labels (710, 712, 714, 716) to the copies (650, 652) included in the respective cluster (126), and a step of optically reading out the labels (710, 712, 714, 716).

15. The method according to claim 14, further comprising a step of physically expanding the biological sample (102) to increase spatial resolution of the optical readout.
